# EUROPEAN PATENT APPLICATION

(11) **EP 4 663 165 A2**
(43) Date of publication of application: **17.12.2025**
(21) Application number: 25211723.9
(22) Date of filing: 10.02.2021
(51) Int. Cl.: A61F 2/24

(54) **PROSTHETIC HEART VALVE**

(30) Priority: 19.02.2020 US 202062978455 P
(62) Divisional of application: 21710668.1
(71) Applicant: Edwards Lifesciences Corporation, Irvine, CA 92614 (US)
(72) Inventor: The designation of the inventor has not yet been filed
(74) Representative: Venner, Julia Ann

(57) **Abstract**

A prosthetic heart valve comprises an annular frame that is radially compressible and expandable between a radially compressed state and a radially expanded state, wherein the frame elongates in an axial direction when radially compressed from the radially expanded state to the radially compressed state. Leaflets are coupled to the frame such that when the frame is in the radially expanded state, the leaflets have slack in an axial direction, wherein when the frame is radially compressed to a partially radially compressed state between the radially expanded state and the radially compressed state, the leaflets are in a relaxed state, and wherein when the frame is in the radially compressed state, the leaflets are stretched in the axial direction.

## Description

### CROSS-REFERENCED TO RELATED APPLICATION

The present application claims the benefit of U.S. Provisional Application No. 62/978,455, filed February 19, 2020. which is incorporated herein by reference.

### FIELD

The present disclosure relates to prosthetic heart valves and to methods for assembling prosthetic heart valves.

### BACKGROUND

The human heart can suffer from various valvular diseases. These valvular diseases can result in significant malfunctioning of the heart and ultimately require repair of the native valve or replacement of the native valve with an artificial valve. There are a number of known repair devices (e.g., stents) and artificial valves, as well as a number of known methods of implanting these devices and valves in humans. Percutaneous and minimally-invasive surgical approaches are used in various procedures to deliver prosthetic medical devices to locations inside the body that are not readily accessible by surgery or where access without surgery is desirable. In one specific example, a prosthetic heart valve can be mounted in a crimped state on the distal end of a delivery device and advanced through the patient's vasculature (e.g., through a femoral artery and the aorta) until the prosthetic valve reaches the implantation site in the heart. The prosthetic valve is then expanded to its functional size, for example, by inflating a balloon on which the prosthetic valve is mounted, actuating a mechanical actuator that applies an expansion force to the prosthetic valve, or by deploying the prosthetic valve from a sheath of the delivery device so that the prosthetic valve can self-expand to its functional size.

Prosthetic valves that rely on a mechanical actuator for expansion can be referred to as "mechanically expandable" prosthetic heart valves. The actuator typically takes the form of pull cables, sutures, wires and/or shafts that are configured to transmit expansion forces from a handle of the delivery apparatus to the prosthetic valve.

Most expandable, transcatheter heart valves comprise a cylindrical metal frame or stent and prosthetic leaflets mounted inside the frame. The leaflets may be attached to the frame along their cusp edge portions and at commissure tabs (also referred to as leaflet tabs) of the leaflets. Mechanically expandable valves, as well as some balloon-expandable valves and self-expandable valves, elongate axially when radially compressed for delivery into a patient. This can cause overstretching of the leaflets in an axial direction, which can deform and/or damage the leaflets.

Accordingly, a need exists for improved prosthetic heart valve leaflet assemblies, and methods for assembling prosthetic heart valves.

### SUMMARY

In a representative embodiment, a prosthetic heart valve comprises an annular frame that is radially compressible and expandable between a radially compressed state and a radially expanded state, wherein the frame elongates in an axial direction when radially compressed from the radially expanded state to the radially compressed state; and a plurality of leaflets disposed inside of the frame and configured to permit blood to flow through the frame in a first direction and block the flow of blood in a second direction, opposite the first direction. The leaflets are coupled to the frame such that when the frame is in the radially expanded state, the leaflets have slack in an axial direction, wherein when the frame is radially compressed to a partially radially compressed state between the radially expanded state and the radially compressed state. the leaflets are in a relaxed state, and wherein when the frame is in the radially compressed state, the leaflets are stretched in the axial direction.

In another representative embodiment, a prosthetic heart valve comprises an annular frame that is radially compressible and expandable between a radially compressed state and a radially expanded state, wherein the frame elongates in an axial direction when radially compressed from the radially expanded state to the radially compressed state; and a leaflet assembly comprising a plurality of leaflets connected to each other at commissures of the leaflet assembly, wherein the leaflets are configured to permit blood to flow through the frame in a first direction and block the flow of blood in a second direction, opposite the first direction. Each commissure is coupled to the frame and each leaflet has a cusp edge portion that is coupled to the frame, and wherein each leaflet has two opposing neck regions unattached to the frame, each neck region extending from one of the commissures to an adjacent end of the cusp edge portion. When the frame is in the radially expanded state, the leaflets have slack in an axial direction in the neck regions of the leaflets, wherein when the frame is radially compressed to a partially radially compressed state between the radially expanded state and the radially compressed state, the leaflets are in a relaxed state, and wherein when the frame is in the radially compressed state, the leaflets are stretched in the axial direction along the neck regions.

In another representative embodiment, a method of assembling a prosthetic heart valve comprises placing a plurality of leaflets in an annular frame that is radially compressible and expandable between a radially compressed state and a radially expanded state, wherein the frame elongates in an axial direction when radially compressed from the radially expanded state to the radially compressed state; placing the frame in a partially radially compressed state between the radially expanded state and the radially compressed state; and coupling the leaflets to the frame while the leaflets are in a relaxed state and the frame is in the partially radially compressed state such that when the frame is expanded to the radially expanded state, the leaflets have slack in an axial direction, and wherein when the frame is compressed to the radially compressed state, the leaflets are stretched in the axial direction.

In another representative embodiment, a prosthetic heart valve comprises an annular frame that is radially compressible and expandable between a radially compressed state and a radially expanded state, the frame comprising a plurality of interconnected struts. A plurality of leaflets is connected to each other to form commissures, wherein the leaflets are configured to permit blood to flow through the frame in a first direction and block the flow of blood in a second direction, opposite the first direction. Each commissure is coupled to the frame and each leaflet has a cusp edge portion that is coupled to the frame, and wherein each leaflet has two opposing neck regions unattached to the frame, each neck region extending from one of the commissures to an adjacent end of the cusp edge portion. The prosthetic heart valve further comprises a plurality of discrete fabric skirts. each being sutured to the cusp edge portion of a leaflet on an outflow surface of the leaflet, wherein each fabric skirt has an extension portion extending beyond the cusp edge portion of the leaflet, the extension portion being connected to one or more of the struts of the frame with sutures; and a plurality of discrete fabric reinforcing strips, each being sutured to the cusp edge portion of a leaflet on an inflow surface of the leaflet.

In another representative embodiment, a method of assembling a prosthetic heart valve comprises forming a plurality of leaflet sub-assemblies, each leaflet sub-assembly including a leaflet and a skirt. wherein each leaflet comprises a cusp edge portion, two opposing commissure tabs, and two opposing neck regions, each neck region extending from one of the commissure tabs to an adjacent end of the cusp edge portion. wherein each leaflet sub-assembly is formed by securing the skirt on an outflow side of the leaflet such that the skirt extends along the cusp edge portion; after forming each leaflet sub-assembly, forming a leaflet assembly by connecting each commissure tab of each leaflet sub-assembly with a commissure tab of another leaflet sub-assembly to form a commissure of the leaflet assembly; and after forming the leaflet assembly, coupling the leaflet assembly to a frame of the prosthetic valve by connecting each commissure to a commissure support portion of the frame and connecting the skirt of each leaflet sub-assembly to struts of the frame, wherein the neck regions remain unattached to the frame.

In another representative embodiment, a method of assembling a prosthetic heart valve comprises placing a plurality of leaflets in an annular frame that is radially compressible and expandable between a radially compressed state and a radially expanded state, wherein the frame elongates in an axial direction when radially compressed from the radially expanded state to the radially compressed state; placing the frame in a partially radially compressed state between the radially expanded state and the radially compressed state; and coupling the leaflets to the frame while the leaflets are in a relaxed state and the frame is in the partially radially compressed state such that when the frame is expanded to the radially expanded state. the leaflets are stretched in a widthwise direction.

The foregoing and other objects, features, and advantages of the invention will become more apparent from the following detailed description, which proceeds with reference to the accompanying figures.

### BRIEF DESCRIPTION OF THE DRAWINGS

FIG. 1 is a perspective view of an exemplary embodiment of a prosthetic heart valve.
FIG. 2 is a plan view of one of the leaflets of the prosthetic valve of FIG. 1 in a laid flat configuration.
FIGS. 3A-3B are side views of the frame of the prosthetic valve of FIG. 1 in a radially compressed state and a radially expanded state, respectively.
FIG. 4 is a plan view of an alternative embodiment of a leaflet for a prosthetic heart valve.
FIG. 5 is a perspective view of a portion of another embodiment of a prosthetic heart valve that includes leaflets of the type shown in FIG. 4.
FIG. 6A is a schematic cross-sectional view of the prosthetic heart valve of FIG. 5 in a radially compressed state.
FIG. 6B is a schematic cross-sectional view of the prosthetic heart valve of FIG. 5 in a partially radially compressed state.
FIG. 6C is a schematic cross-sectional view of the prosthetic heart valve of FIG. 5 in a radially expanded state.
FIGS. 7A-7B are top plan views of the prosthetic heart valve of FIG. 5 shown with the leaflets in the open position and the closed position, respectively.
FIG. 8 shows a leaflet sub-assembly comprising a leaflet and a discrete skirt secured to the leaflet.
FIG. 9 shows a reinforcing strip for the leaflet sub-assembly of FIG. 8.
FIG. 10 is a perspective view of the leaflet sub-assembly of FIG. 8 after attaching the reinforcing strip to the leaflet.
FIG. 11 is a perspective view of a portion of another embodiment of a prosthetic heart valve that includes leaflet sub-assemblies of the type shown in FIG. 10.
FIG. 12 is an enlarged, cross-sectional view of a portion of the prosthetic heart valve of FIG. 11.
FIG. 13 shows a delivery apparatus adapted to deliver a prosthetic heart valve.

### DETAILED DESCRIPTION

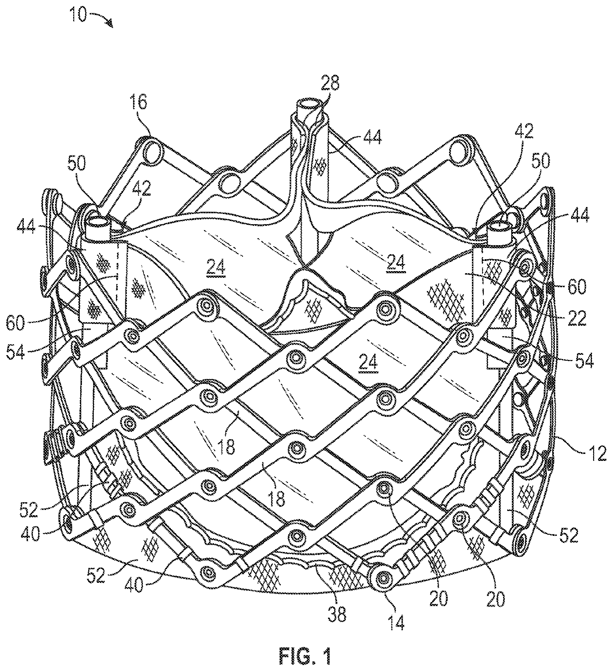

FIG. 1 shows an exemplary prosthetic heart valve 10, according to one embodiment. The prosthetic heart valve 10 can be radially compressible and expandable between a radially compressed configuration for delivery into a patient and a radially expanded configuration. In particular embodiments, the prosthetic heart valve 10 can be implanted within the native aortic annulus, although it also can be implanted at other locations in the heart, including within the native mitral valve, the native pulmonary valve, and the native tricuspid valve. The prosthetic heart valve 10 can include an annular stent or frame 12 having a first end 14 and a second end 16.

In the depicted embodiment, the first end 14 is an inflow end and the second end 16 is an outflow end. The outflow end 16 can be coupled to a delivery apparatus for delivering and implanting the prosthetic heart valve within the native aortic valve is a transfemoral, retrograde delivery approach. Thus, in the delivery configuration of the prosthetic heart valve, the outflow end 16 is the proximal-most end of the prosthetic valve. In other embodiments, the inflow end 14 can be coupled to the delivery apparatus, depending on the particular native valve being replaced and the delivery technique that is used (e.g., trans-septal, transapical, etc.). For example, the inflow end 14 can be coupled to the delivery apparatus (and therefore is the proximal-most end of the prosthetic heart valve in the delivery configuration) when delivering the prosthetic heart valve to the native mitral valve via a trans-septal delivery approach.

The frame 12 can be made of any of various suitable materials, such as stainless steel, a cobalt chromium alloy, or a nickel titanium alloy ("NiTi"), for example Nitinol. Referring again to FIG. 1, as shown, the frame 12 can include a plurality of interconnected struts 18 arranged in a lattice-type pattern. The struts 18 are shown as positioned diagonally, or offset at an angle relative to, and radially offset from, a longitudinal axis of the prosthetic heart valve 10 when the prosthetic heart valve 10 is in the expanded configuration. In other implementations, the struts 18 can be offset by a different amount than depicted in FIG. 1, or some or all of the struts 18 can be positioned parallel to the longitudinal axis of the prosthetic heart valve 10.

In the illustrated embodiment, the struts 18 are pivotably coupled to one another at one or more pivot joints along the length of each strut. For example, in the illustrated configuration, each of the struts 18 can be formed with apertures at opposing ends of the strut and apertures spaced along the length of the strut. Respective hinges can be formed at the locations where struts 18 overlap each other via fasteners or pivot members, such as rivets or pins 20 that extend through the apertures. The hinges can allow the struts 18 to pivot relative to one another as the frame 12 is radially expanded or compressed. such as during assembly, preparation, or implantation of the prosthetic heart valve 10.

In some embodiments, the frame 12 can be constructed by forming individual components (e.g., the struts and fasteners of the frame) and then mechanically assembling and connecting the individual components together. In other embodiments, the struts 28 are not coupled to each other with respective hinges but are otherwise pivotable or bendable relative to each other to permit radial expansion and contraction of the frame 12. For example, the frame 12 can be formed (e.g., via laser cutting, electroforming or physical vapor deposition) from a single piece of material (e.g., a metal tube). Further details regarding the construction of the frame and the prosthetic heart valve are described in U.S. Patent Application Publication Nos. 2018/0153689, 2018/0344456, and 2019/0060057, all of which are incorporated herein by reference.

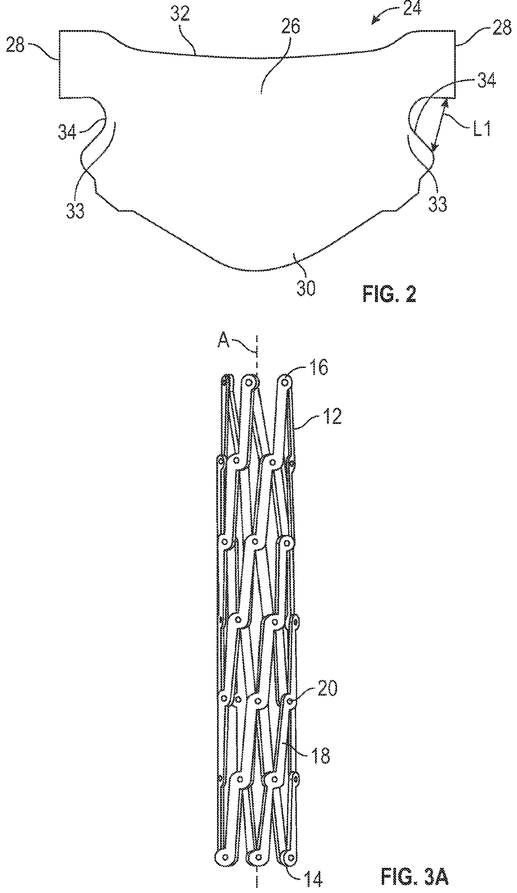

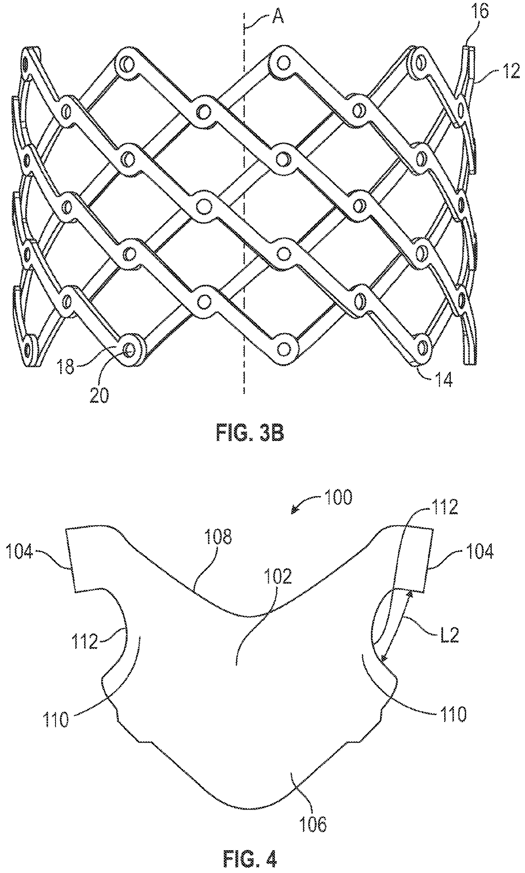

The frame 12 can be radially compressed and expanded between a radially compressed state and a radially expanded state. FIG. 3A shows the bare frame 12 (without other components of the prosthetic valve) is the radially compressed state for delivery into a patient. FIG. 3B shows the bare frame 12 in the radially expanded state, such as when expanded inside the patient's body at a desired implantation site. As shown in FIGS. 3A and 3B, the frame 12 elongates axially along a central longitudinal axis A when the frame is radially compressed.

The prosthetic heart valve 10 can also include a valvular structure 22 which is coupled to the frame 12 and configured to regulate the flow of blood through the prosthetic heart valve 10 from the inflow end 14 to the outflow end 16. The valvular structure 22 can include, for example, a leaflet assembly comprising one or more leaflets 24 (three leaflets 24 in the illustrated embodiment) made of a flexible material. The leaflets 24 of the leaflet assembly can be made from in whole or part, biological material, bio-compatible synthetic materials, or other such materials. Suitable biological material can include, for example, bovine pericardium (or pericardium from other sources).

As best shown in FIG. 2, each leaflet 24 in the illustrated embodiment includes a main body 26 (also referred to as a "belly" portion of the leaflet), and two opposing commissure tabs 28 arranged on opposite sides of the main body 26. The main body 26 has a cusp edge portion 30 (also referred to as the inflow edge portion of the leaflet) and a free edge portion 32 that can coapt with the free edge portions of adjacent leaflets when the leaflet assembly is in a closed position under pressure from the backflow of blood. As further shown in FIG. 2, in particular embodiments, the main body 26 of each leaflet 24 optionally can include a notch or indention 34 on each side of the leaflet between each commissure tab 28 and an adjacent upper end of the cusp edge portion 30. The portions of the main body containing the notches 34 can be referred to as "neck" regions 33 of the leaflet.

The leaflets 24 can be mounted to the frame in a variety of ways. In the illustrated embodiment, for example, the cusp edge portion 30 of each leaflet can be sutured to an inner skirt 36 along a stitch line 38 (sometimes referred to as a "scallop" line). The stitch line 38 can be formed by forming in-an-out stitches through the cusp edge portions of the leaflets and the skirt with one or more sutures, such as an Ethibond suture. The skirt 36 in turn can be connected to the frame 12 with sutures 40 that extend through the skirt and around selected struts 18 of the frame 12.

Each commissure tab 28 of a leaflet 24 can be paired with an adjacent commissure 28 of an adjacent leaflet 24 to form a commissure 42. The commissures 42 can be mounted to respective commissure support portions of the frame 12. In some embodiments, as shown in FIG. 1, the commissures 42 can be mounted (e.g., sutured) directly to commissure support portions of actuators 50 of the frame 12 via commissure attachment members 44, which can be pieces of relatively tear resistant material, such as a fabric. As one example, as depicted in FIG. 1, at each commissure 42, the commissure tabs 28 can be at least partially wrapped around opposite sides of the commissure support portion and the commissure attachment member 44 can be wrapped around the commissure tabs 28 and the commissure support portion. The commissure attachment member 44 can be stitched to the commissure tabs 28 with sutures 60, thereby securing the commissure 42 to the commissure support portion of the actuator 50.

The neck regions 33 of the leaflets can remain unattached to the frame to promote stretching of the leaflets in the axial direction (along longitudinal axis A) when the frame 12 is radially compressed. The notches 34 are shaped to promote stretching of the leaflets in a radial direction when the frame 12 is radially expanded to its functional size.

In other embodiments, the commissures 42 can be mounted to support struts or posts of the frame that are separate from the actuators 50. In still other embodiments, the commissures 42 can be secured to support struts or posts that are integral components of the frame. Further details regarding transcatheter prosthetic heart valves, including the manner in which the valvular structure can be coupled to the frame 12 of the prosthetic heart valve 10, can be found, for example, in U.S. Patent Nos. 6,730,118, 7,393,360, 7,510,575, 7,993,394, and 8,652,202, and U.S. Patent Application Publication No. 2018/0325665, all of which are incorporated herein by reference in their entireties.

As noted above, the prosthetic heart valve 10 can further include a plurality of actuators 50, which are mounted to and equally spaced around the inner surface of the frame 12. The actuators are configured to apply expansion and compression forces to the frame for radially expanding and compressing the prosthetic valve.

In the illustrated embodiment, the actuators 50 are linear actuators, each of which comprises an inner member, or piston, 52 and an outer member, or cylinder, 54. The inner member 52 is pivotably coupled to a junction of the frame, such as at the first end 14, while the outer member 54 is pivotably coupled to another junction of the frame closer to the second end 16. Moving the inner member 52 proximally relative to the outer member 54 and/or moving the outer member 54 distally relative to the inner member 52 is effective to radially expand the prosthetic valve. Conversely, moving the inner member 52 distally relative to the outer member 54 and/or moving the outer member 54 proximally relative to the inner member 52 is effective to radially compress the prosthetic valve. The actuators 50 can include locking mechanisms, such as ratchet mechanisms, that are configured to retain the prosthetic valve in an expanded state inside the patient's body.

In some embodiments, each of the actuators 50 can be configured to form a releasable connection with one or more respective actuators of a delivery apparatus of a transcatheter delivery system. The actuators of the delivery apparatus can transmit forces from a handle of the delivery apparatus to the actuators 50 for expanding or compressing the prosthetic valve. Further details of the actuators, locking mechanisms and delivery apparatuses for actuating the actuators can be found in U.S. Patent Application Publication Nos. 2018/0153689, 2019/0060057 and 2018/0325665, each of which is incorporated herein by reference in its entirety. Any of the actuators and locking mechanisms disclosed in the previously filed applications can be incorporated in any of the prosthetic valves disclosed herein. Further, any of the delivery apparatuses disclosed in the previously filed applications can be used to deliver and implant any of the prosthetic valves discloses herein.

As noted above, each of the actuators 50 can be used to support a respective commissure 42 of the leaflet assembly. In the illustrated embodiment, for example, the commissures 42 are mounted on the outer members 54 of the actuators, which serve as commissure support portions of the prosthetic valve.

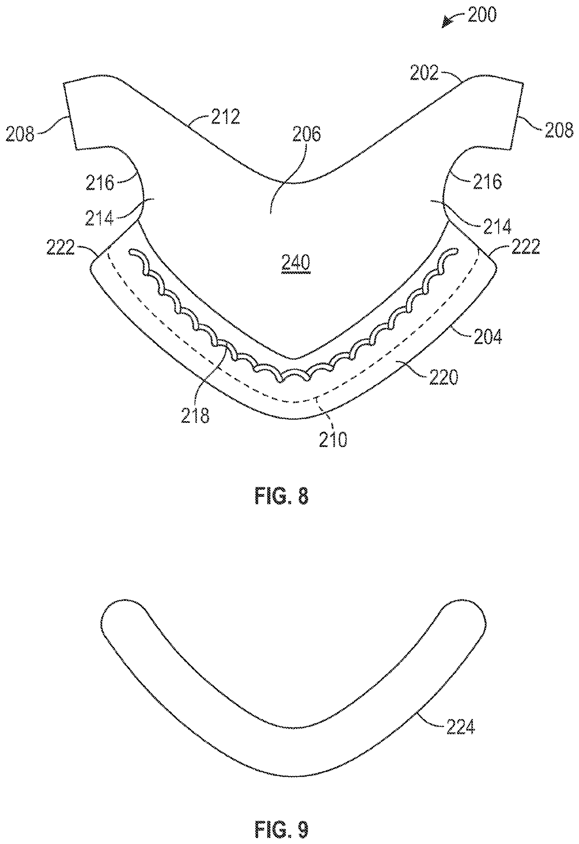

The prosthetic heart valve 10 can also include one or more skirts or sealing members. For example, as shown in FIG. 1, the prosthetic heart valve 10 can include an inner skirt 36 mounted on the inner surface of the frame 12. As shown in FIG. 1, the inner skirt 36 is a circumferential inner skirt that spans an entire circumference of the inner surface of the frame 12. The inner skirt 36 can function as a sealing member to prevent or decrease perivalvular leakage (e.g., when the valve is placed at the implantation site) and as an attachment surface to anchor the leaflets 24 to the frame 12. For example, as discussed above, the cusp edge portions 30 of the leaflets 24 can be sutured directly to the inner skirt 36, which in turn can be directly connected to selected struts 18 of the frame, such as with sutures 40, as shown in FIG. 1. In alternative embodiments, each leaflet 24 can be sutured to a discrete skirt, which is turn is connected to selected struts 18 of the frame. as further described below in connection with FIGS. 8-12).

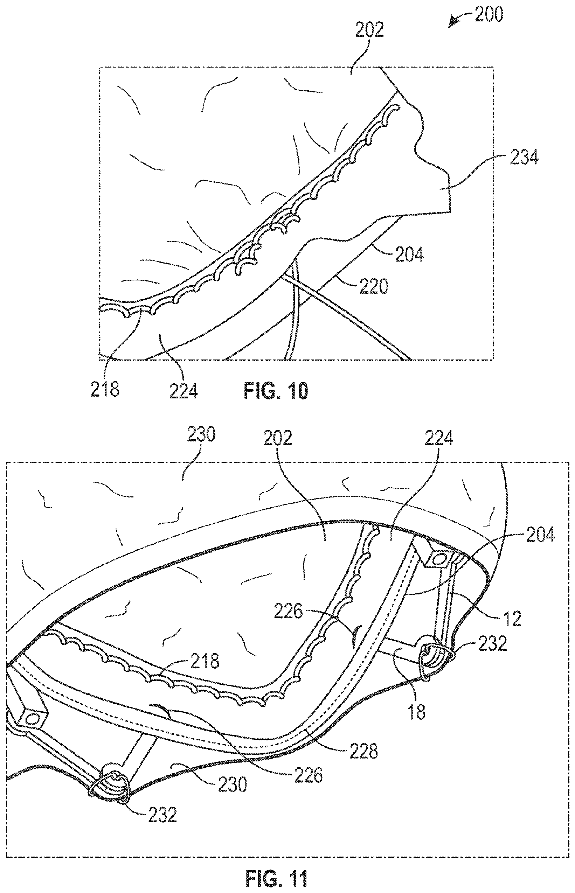

The prosthetic heart valve 10 can also include an outer skirt mounted on the outer surface of the frame 12 (not shown in FIG. 1; see. e.g.. outer skirt 230 in FIG. 11). The outer skirt can function as a sealing member for the prosthetic valve by sealing against the tissue of the native valve annulus and helping to reduce paravalvular leakage past the prosthetic valve. The inner and outer skirts can be formed from any of various suitable biocompatible materials, including any of various synthetic materials (e.g., polyethylene terephthalate (PET)) or natural tissue (e.g., pericardial tissue). In particular embodiments, the skirts can be formed from any of various biocompatible fabrics (e.g., a PET fabric). which can have woven, braided, or knitted construction or combinations thereof. The inner and outer skirts can be mounted to the frame using sutures, an adhesive, welding, and/or other means for attaching the skirts to the frame.

FIG. 2 shows a leaflet 24 in a non-deformed, or "free state", meaning that the leaflet is not stretched axially or radially. Typically, the leaflets of a conventional prosthetic valve are sized and shaped relative to the frame to assume a non-deformed state when the frame is in a radially expanded state, which is desirable to promote coaptation of the free edge portions 32 of the leaflets 24 when the leaflet assembly is in a closed state blocking the flow of the blood through the prosthetic valve. In some cases, the frame elongates axially when the frame is radially compressed from the radially expanded state to the radially compressed state, thereby stretching the leaflets in the axial direction. As depicted in FIGS. 3A-3B, in some embodiments, the length of the frame 12 can increase by 100% when radially compressed from the fully expanded state (FIG. 3B) to the fully compressed state (FIG. 3A). This increase in the length of the frame can overstretch the leaflets, which can damage the leaflets.

Thus, in particular embodiments, the leaflets 24 can be sized and shaped relative to the frame 12 to assume a non-deformed or free state when the frame is in an intermediate state between the fully compressed state and the fully expanded state. The intermediate state can be referred to as a partially radially compressed state of the frame. Advantageously, this reduces stretching of the leaflets in the axial direction when the frame is radially compressed to the fully compressed state. In particular, the amount of stretching of the neck regions 33 (which are unattached to the frame) in the axial direction is reduced, thereby avoiding or minimizing damage to the leaflets when the frame is radially compressed.

In particular embodiments, a prosthetic valve. such as prosthetic valve 10, can include a frame of a first size (equal to an expanded diameter of the frame) and leaflets that are designed for a frame of a second, smaller size (equal to an expanded diameter of the frame), which allows the leaflets to assume a free state when the frame is in a partially radially compressed state. In other words, the leaflets designed for a prosthetic valve of a first size (e.g., 23 mm) can be assembled in a frame of a prosthetic valve of a second, larger size (e.g., 29 mm).

FIG. 4 shows an example of a leaflet 100 that has a slightly different configuration than the leaflet 24 and is designed for a relatively smaller size prosthetic valve. Like leaflet 24, the leaflet 100 has a main body 102, commissure tabs 104, a cusp edge portion 106, a free edge portion 108, and neck regions 110, which can define notches 112. Because the leaflet 100 is designed for a relatively smaller size valve, the notches 112 of neck regions 110 can have a length L2 that is longer than a length L1 of notches 34 of neck regions 33 of the leaflet 24. Further, the free edge portion 108 of the leaflet 100 can a more pronounced V-shape compared to the free edge portion 32 of the leaflet 24.

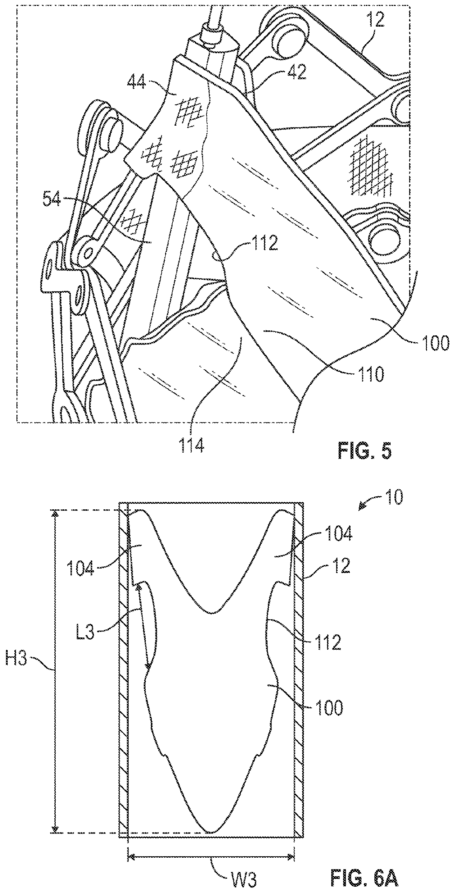

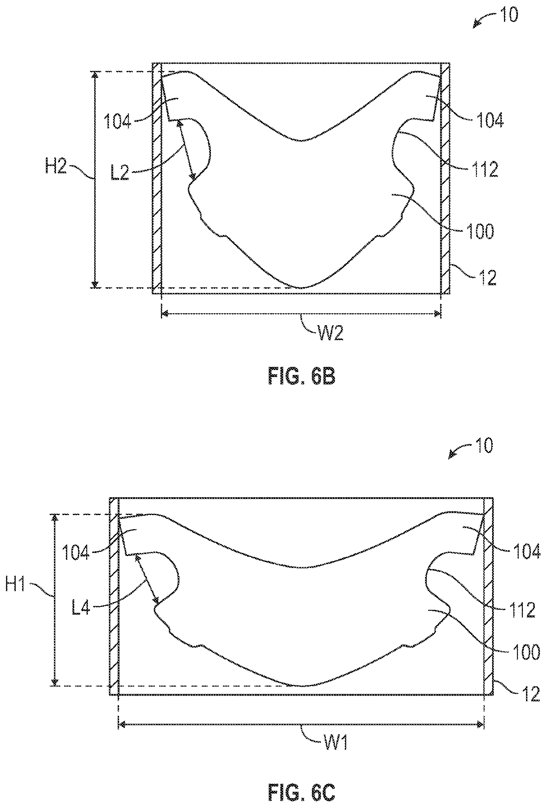

In some embodiments, as shown in FIG. 5, a prosthetic valve 10 can include a plurality of leaflets 100 (e.g., three) instead of leaflets 24. FIGS. 6A-6C are schematic representations of the prosthetic valve 10 with leaflets 100 (one which is shown for purposes of illustration) in various stages of compression. FIG. 6A shows the prosthetic valve 10 in a fully radially compressed state. FIG. 6B shows the prosthetic valve 10 is a partially radially compressed state. FIG. 6C show the prosthetic valve 10 in a fully expanded state.

The leaflets 100 are in a free state in the partially radially compressed state of FIG. 6C. Thus, when the prosthetic valve 10 is in the fully expanded state (FIG. 6C), the leaflets 100 have a height H1 (measured from the inflow-most location of the leaflet to the outflow-most location of the leaflet) and a width W1 (measured from the radial outermost edge of a commissure tab 104 to the same location on the opposing tab 104). When the prosthetic valve 10 is in the partially compressed state (FIG. 6B), the leaflets 100 have a height H2 that is greater than H1 and width W2 that is less than W1. When the prosthetic valve is in the fully compressed state (FIG. 6A), the leaflets 100 have a height H3 that is greater than H2 and a width W3 that is less than W2. Further, the length of the notches 112 increase from L2 in the partially compressed state to L3 in the fully compressed state. The length of notches 112 can decrease from L2 in the partially compressed state to L4 in the fully expanded state.

As best shown in FIG. 5, when the prosthetic valve 10 is in the fully expanded state, slack 114 can form along the neck regions 110 as a result of the decreased height H1 of the leaflet. The slack 114 forms in an axial direction, meaning that portions of the leaflet are brought closer together in an axial direction to form the slack. When the portions of the leaflets are brought closer together, they can a radially extending fold in the neck regions 110. Moreover, when the prosthetic valve 10 is in the fully expanded state, the leaflets 100 can be stretched in a transverse plane perpendicular to the central longitudinal axis of the prosthetic valve. In other words, the width W1 of the leaflet 100 (FIG. 6C) can represent the width of the leaflet that is stretched in a widthwise direction of the leaflet extending from one commissure tab 104 to the other commissure 104.

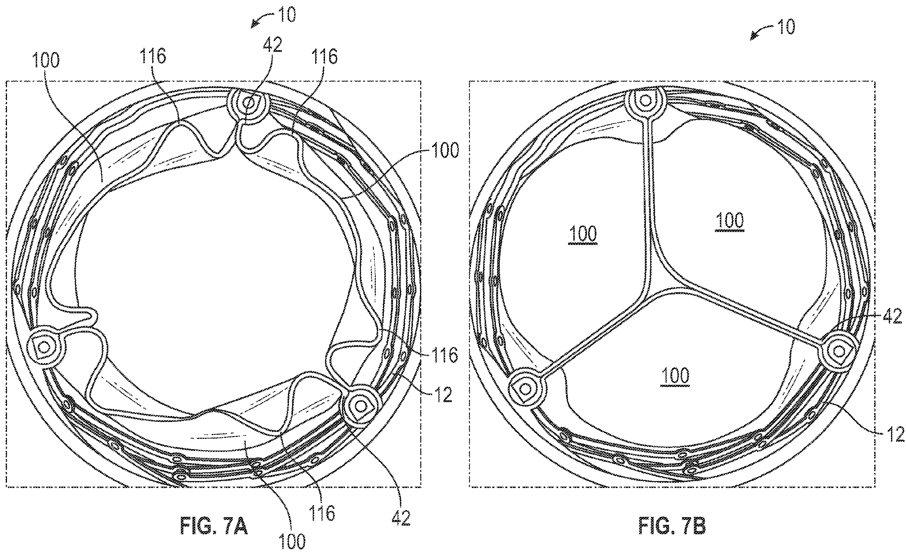

FIGS. 7A and 7B show the prosthetic valve 10 with leaflets 100 in an open state and a closed state, respectively. In the open state of the leaflets (FIG. 7A), slack 116 can form along the free edge portions of the leaflets 100 in the vicinity of the commissures 42. The slack 116 can form axially extending folds extending downwardly from the free edge portions of the leaflets.

In one specific implementation, the prosthetic valve 10 can include a frame 12 for a 29-mm valve (the size of the valve being the outer diameter of the frame in the radially expanded state) and leaflets 100 for a 23-mm valve. The notches 112 of the leaflets 100 can have a length L2 of about 7 mm in its free state (FIG. 6B), a length L3 of about 10 mm when the frame is fully radially compressed (FIG. 6A), and a length L4 of about 5 mm to 7 mm when the frame is fully radially expanded (FIG. 6C). Comparatively, the same prosthetic valve 10 having leaflets 24 sized for a 29-mm valve (where the leaflets are in a free state when the frame is fully radially expanded), the notches 34 stretch axially from a length L1 of about 5 mm when the frame is fully expanded to about 10 mm when the frame is fully compressed. As such, it can be appreciated that the leaflets 100 undergo less stretching in an axial direction when the frame is fully radially compressed than leaflets 24.

It should be noted that leaflet 100 is one example of a leaflet that can be used in the prosthetic valve 10 to reduce axial stretching of the leaflet when the frame is fully radially compressed. In particular, the prosthetic valve can have leaflets that have the same overall shape of leaflets 24 (or other shapes) but are otherwise sized such that the leaflets are in a free state when the frame is in a partially radially compressed state.

As discussed above, in the embodiment of FIG. 1, the cusp edge portions 30 of the leaflets 24 are sutured to an inner skirt 36. Since the inner skirt 36 spans the entire circumference of the inner surface of the frame 12, attachment of numerous leaflets (for example, three leaflets) 24 to the inner skirt 36 is time consuming and requires delicate assembly skills to avoid defects that might occur through the assembly process.

Thus, it may be desirable to assemble each leaflet of a leaflet assembly separately, with an individual or discrete skirt and leaflet. For example, a leaflet assembly may include a plurality of separately formed leaflet sub-assemblies, each leaflet sub-assembly including a skirt and leaflet secured to one another along a cusp edge portion of the leaflet. In some embodiments, the skirt and an additional reinforcing element may form a supporting structure for the leaflet. Each individual leaflet sub-assembly may then be connected to additional leaflet sub-assemblies at their commissure tabs to form commissures. The leaflet assembly can then be placed inside a frame. The skirt and optionally the reinforcing element of each of each leaflet sub-assembly can be connected (e.g., via sutures) to struts of the frame and/or an outer skirt. The commissures can be connected to respective commissure support members (e.g., actuators 50) as disclosed herein. Such an assembly process may allow each leaflet sub-assembly to be assembled more quickly and easily, on a relatively flat, two-dimensional platform, prior to being attached to the three-dimensional frame of the prosthetic heart valve.

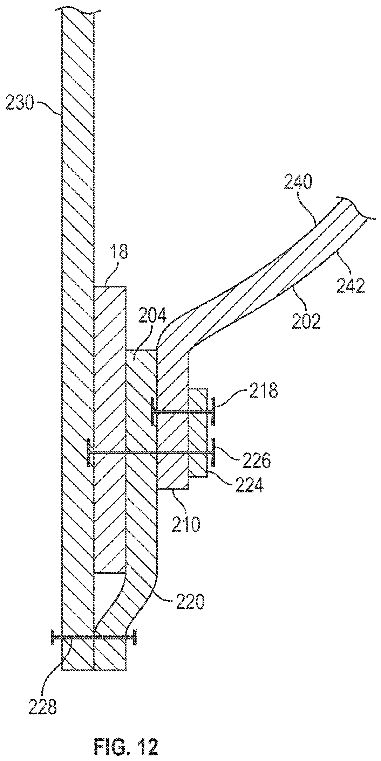

FIG. 8 shows a leaflet sub-assembly 200, according to one embodiment, that can be used in the assembly of a prosthetic valve, such as prosthetic valve 10. The leaflet sub-assembly 200 in the illustrated embodiment includes a leaflet 202 and a discrete skirt 204. Like leaflets 24 and 100, the leaflet 202 has a main body 206, commissure tabs 208, a cusp edge portion 210, a free edge portion 212, and neck regions 214, which can define notches 216. The leaflet 202 has first and second major surfaces 240. 242, respectively (see FIG. 12). When assembled in a frame, the first major surface 240 is an outwardly facing surface and also the outflow surface or outflow side of the leaflet. The second major surface 242 is an inwardly facing surface and also the inflow surface or inflow side of the leaflet.

The skirt 204 can be connected to the outwardly facing surface 240 of the cusp edge portion 210 with a suture 206 which can track the curvature of the cusp edge portion 210 and the skirt 204. A lower portion of the skirt 204 can extend beyond the cusp edge portion 210 of the leaflet to form an extended portion 220 which can be used to connect the skirt 204 to other components of the prosthetic valve. As further shown in FIG. 8, the skirt 204 desirably is sized and shaped such that opposite ends 222 of the skirt 204 are aligned or substantially aligned with the opposite ends of the cusp edge portion 210 of the leaflet and do not extend into the notches 216. In this manner, the skirt 204 does not inhibit the leaflet from stretching in the axial direction along the neck regions 214 when the frame 12 is radially compressed to the radially compressed state.

An exemplary method for assembling a prosthetic heart valve, such as prosthetic valve 10 is as follows. A plurality of leaflet sub-assemblies 200 can be formed in the manner described above and shown in FIG. 8 for each leaflet of the prosthetic valve. For example, three leaflet sub-assemblies 200 can be formed if the prosthetic valve is to have three leaflets 202. Each leaflet sub-assembly 200 can then be positioned into a frame 12. The skirt 204 of each leaflet sub-assembly 200 can be connected to the frame 12, such as by suturing each skirt 204 to selected struts 18 (similar to the way skirt 36 is sutured to struts 18) and/or suturing the skirt 204 to an outer skirt (such as outer skirt 230, described below).

The commissures tabs 208 can be connected to adjacent commissure tabs 208 of adjacent leaflets to form commissures (similar to commissures 42), which are then connected to respective commissure support members of the frame, such as actuators 50. Commissure attachment members (e.g., attachment members 44) can be used to attach the commissures to the actuators 50 or other commissure support members of the frame. In some embodiments, the commissures are at least partially assembled or fully assembled prior to placing the leaflet sub-assemblies 200 inside the fame and connecting the skirts 204 to the frame, such as disclosed in U.S. Provisional Application No. 62/928.993. filed October 31, 2019. which is incorporated herein by reference.

Referring to FIGS. 9-10, in some embodiments, each leaflet sub-assembly 200 can further include a discrete reinforcing strip 224 secured to the inwardly facing surface 242 of the cusp edge portion 210, on the opposite side of the leaflet from the skirt 204. The reinforcing strip 224 can be secured to the leaflet 202 and the skirt 204 with the suture 218. The opposing end portions of the reinforcing strip 224 can include projections or wing portions 234 (one of which is shown in FIG. 10). The wing portions 234 can be secured to respective actuators (e.g., actuators 50 of FIG. 1), such as with sutures, to help seal the sub-commissure area of the leaflet assembly during valve cycling.

The skirts 204 and reinforcing strips 224 can be formed from any of various suitable biocompatible materials, including any of various synthetic materials (e.g., (PET) or natural tissue (e.g., pericardial tissue). In particular embodiments, the skirts 204 and reinforcing strips 224 can be formed from any of various biocompatible fabrics (e.g., a PET fabric), which can have woven, braided, or knitted construction or combinations thereof.

Referring now to FIGS. 11-12, after forming each leaflet sub-assembly 200 including a reinforcing strip 224, and optionally pre-assembling the commissures, the cusp edge portions of each sub-assembly 200 can be connected to selected struts 18 of the frame 12 with sutures 226, each of which can extend through the skirt 204, the cusp edge portion 210 of the leaflet, and the reinforcing strip 224 and around a selected strut 18. In some embodiments, one or more of the sutures 226 can be placed through the skirt 204 around a selected strut 18 while avoiding the cusp edge portion 210 and the reinforcing strip 224.

In particular embodiments, as shown in FIGS. 11-12, each skirt 204 can be further connected to an outer skirt 230 of the prosthetic valve with one or more sutures 228. The sutures 228 can be attached in the form of in-and-out stitches that extend through the skirts 204 and the outer skirt 230 along an undulating stitch line that tracks the curvature of the skirts 204. The outer skirt 230 can be an annular skirt that extends completely around an outer surface of the frame 12. The outer skirt 230 can be connected to the frame 12, such as with sutures 232 that extend around selected struts and through the outer skirt 230. Further details regarding the outer skirt are disclosed in U.S. Application No. 16/252.890 and 62/797,837, which are incorporated herein by reference.

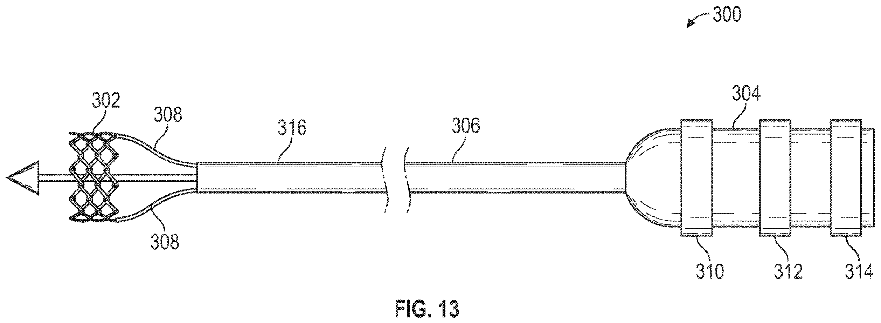

FIG. 13 illustrates a delivery apparatus 300, according to one embodiment, adapted to deliver a prosthetic heart valve 302, such as the illustrated prosthetic heart valve 10, described above. The prosthetic valve 302 can be releasably coupled to the delivery apparatus 300. It should be understood that the delivery apparatus 300 and other delivery apparatuses disclosed herein can be used to implant prosthetic devices other than prosthetic valves, such as stents or grafts.

The delivery apparatus 300 in the illustrated embodiment generally includes a handle 304, a first elongated shaft 306 (which comprises an outer shaft in the illustrated embodiment) extending distally from the handle 304, at least one actuator assembly 308 extending distally through the outer shaft 306. The at least one actuator assembly 308 can be configured to radially expand and/or radially collapse the prosthetic valve 302 when actuated.

Though the illustrated embodiment shows two actuator assemblies 308 for purposes of illustration, it should be understood that one actuator 308 can be provided for each actuator on the prosthetic valve. For example, three actuator assemblies 308 can be provided for a prosthetic valve having three actuators (e.g., actuators 50). In other embodiments, a greater or fewer number of actuator assemblies can be present.

In some embodiments, a distal end portion 316 of the shaft 306 can be sized to house the prosthetic valve in its radially compressed, delivery state during delivery of the prosthetic valve through the patient's vasculature. In this manner, the distal end portion 316 functions as a delivery sheath or capsule for the prosthetic valve during delivery,

The actuator assemblies 308 can be releasably coupled to the prosthetic valve 302. For example, in the illustrated embodiment, each actuator assembly 308 can be coupled to a respective actuator (e.g., actuator 50) of the prosthetic valve 302. Each actuator assembly 308 can comprise a support tube, an actuator member, and a locking tool. The actuator member can extend through the support tube. The support tube has a distal end portion that can be coupled to the outer member 54 of a respective actuator 50, and the actuator member has a distal end portion that can be coupled to the inner member 52 of the respective actuator 50. When actuated, the actuator assembly 308 can transmit pushing and/or pulling forces to the inner and outer members 52, 54 to radially expand and collapse the prosthetic valve as previously described. The actuator assemblies 308 can be at least partially disposed radially within, and extend axially through, one or more lumens of the outer shaft 306. For example, the actuator assemblies 308 can extend through a central lumen of the shaft 306 or through separate respective lumens formed in the shaft 306.

The handle 304 of the delivery apparatus 300 can include one or more control mechanisms (e.g., knobs or other actuating mechanisms) for controlling different components of the delivery apparatus 300 in order to expand and/or deploy the prosthetic valve 302. For example, in the illustrated embodiment the handle 304 comprises first, second, and third knobs 310, 312, and 314.

The first knob 310 can be a rotatable knob configured to produce axial movement of the outer shaft 306 relative to the prosthetic valve 302 in the distal and/or proximal directions in order to deploy the prosthetic valve from the delivery sheath 316 once the prosthetic valve has been advanced to a location at or adjacent the desired implantation location with the patient's body. For example, rotation of the first knob 310 in a first direction (e.g., clockwise) can retract the sheath 316 proximally relative to the prosthetic valve 302 and rotation of the first knob 310 in a second direction (e.g., counter-clockwise) can advance the sheath 316 distally. In other embodiments, the first knob 310 can be actuated by sliding or moving the knob 310 axially, such as pulling and/or pushing the knob. In other embodiments, actuation of the first knob 310 (rotation or sliding movement of the knob 310) can produce axial movement of the actuator assemblies 308 (and therefore the prosthetic valve 302) relative to the delivery sheath 316 to advance the prosthetic valve distally from the sheath 316.

The second knob 312 can be a rotatable knob configured to produce radial expansion and/or contraction of the prosthetic valve 302. For example, rotation of the second knob 312 can move the actuator member and the support tube axially relative to one another. Rotation of the second knob 312 in a first direction (e.g., clockwise) can radially expand the prosthetic valve 302 and rotation of the second knob 112 in a second direction (e.g., counter-clockwise) can radially collapse the prosthetic valve 302. In other embodiments, the second knob 312 can be actuated by sliding or moving the knob 312 axially, such as pulling and/or pushing the knob.

The third knob 314 can be a rotatable knob configured to retain the prosthetic heart valve 302 in its expanded configuration. For example, the third knob 314 can be operatively connected to a proximal end portion of the locking tool of each actuator assembly 308. Rotation of the third knob in a first direction (e.g., clockwise) can rotate each locking tool to advance the locking nuts to their distal positions to resist radial compression of the frame of the prosthetic valve, as described above. Rotation of the knob 314 in the opposite direction (e.g., counterclockwise) can rotate each locking tool in the opposite direction to decouple each locking tool from the prosthetic valve 302. In other embodiments, the third knob 314 can be actuated by sliding or moving the third knob 314 axially, such as pulling and/or pushing the knob.

Although not shown, the handle 304 can include a fourth rotatable knob operative connected to a proximal end portion of each actuator member. The fourth knob can be configured to rotate each actuator member, upon rotation of the knob, to unscrew each actuator member from the proximal portion of a respective actuator. As described above, once the locking tools and the actuator members are uncoupled from the prosthetic valve 302, they can be removed from the patient.

Other delivery devices can be used to deliver and implant the prosthetic valves disclosed herein, such as those disclosed in PCT Application No. PCT/US2020/063104, filed December 3, 2020, and U.S. Application No. 62/990,299, filed March 16, 2020, which are incorporated herein by reference.

A method of delivering prosthetic valve 302 generally includes placing prosthetic valve 302 in a radially compressed state, e.g., by operating the actuators 50 of the prosthetic valve to place the frame in a radially compressed configuration. This can be accomplished by releasably coupling the prosthetic valve to the actuator assemblies 308 of the delivery device and actuating the second knob 312. The radially compressed prosthetic valve optionally can be placed within a sheath 316 of the delivery device. The delivery device and prosthetic device can be advanced over a guidewire through the vasculature of a patient to a selected implantation site (e.g., the native aortic annulus). For example, when implanting the prosthetic valve within the native aortic valve, the delivery device and prosthetic valve can be inserted into and through a femoral artery, and through the aorta to the native aortic valve. At the implantation site, if initially contained within a sheath 316, the prosthetic valve 302 can be deployed from the sheath 316 by actuating the first knob 310. The prosthetic valve 302 can then be radially expanded to a desired size by actuating the second knob 312. Once the prosthetic device is at the desired diameter, the third knob 314 can be actuated to lock the prosthetic valve at the desired diameter. Thereafter, the actuator assemblies of the delivery device can uncoupled from the prosthetic valve by actuating the fourth knob, allowing removal of the delivery device from the patient's body.

### General Considerations

For purposes of this description, certain aspects, advantages, and novel features of the embodiments of this disclosure are described herein. The disclosed methods. apparatus, and systems should not be construed as being limiting in any way. Instead, the present disclosure is directed toward all novel and nonobvious features and aspects of the various disclosed embodiments, alone and in various combinations and sub-combinations with one another. The methods, apparatus, and systems are not limited to any specific aspect or feature or combination thereof, nor do the disclosed embodiments require that any one or more specific advantages be present or problems be solved. The technologies from any example can be combined with the technologies described in any one or more of the other examples. In view of the many possible embodiments to which the principles of the disclosed technology may be applied, it should be recognized that the illustrated embodiments are only preferred examples and should not be taken as limiting the scope of the disclosed technology.

Although the operations of some of the disclosed embodiments are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used herein, with reference to the prosthetic heart valve and the delivery device or apparatus, "proximal" refers to a position, direction. or portion of a component that is closer to the user and a handle of the delivery apparatus that is outside the patient, while "distal" refers to a position, direction, or portion of a component that is further away from the user and the handle and closer to the implantation site. The terms "longitudinal" and "axial" refer to an axis extending in the proximal and distal directions, unless otherwise expressly defined. Further, the term "radial" refers to a direction that is arranged perpendicular to the axis and points along a radius from a center of an object (where the axis is positioned at the center. such as the longitudinal axis of the prosthetic valve).

As used in this application and in the claims, the singular forms "a," "an." and "the" include the plural forms unless the context clearly dictates otherwise. Additionally, the term "includes" means "comprises." Further, the terms "coupled" and "connected" generally mean electrically, electromagnetically, and/or physically (e.g., mechanically or chemically) coupled or linked and does not exclude the presence of intermediate elements between the coupled or associated items absent specific contrary language.

Directions and other relative references (e.g., inner, outer, upper, lower, etc.) may be used to facilitate discussion of the drawings and principles herein, but are not intended to be limiting. For example, certain terms may be used such as "inside," "outside,", "top," "down," "interior," "exterior," and the like. Such terms are used, where applicable, to provide some clarity of description when dealing with relative relationships, particularly with respect to the illustrated embodiments. Such terms are not, however, intended to imply absolute relationships, positions, and/or orientations. For example, with respect to an object, an "upper" part can become a "lower" part simply by turning the object over. Nevertheless, it is still the same part and the object remains the same. As used herein, "and/or" means "and" or "or," as well as "and" and "or."

### Additional Examples of the Disclosed Technology

In view of the above described implementations of the disclosed subject matter, this application discloses the additional examples enumerated below. It should be noted that one feature of an example in isolation or more than one feature of the example taken in combination and, optionally, in combination with one or more features of one or more further examples are further examples also falling within the disclosure of this application.

Example 1: A prosthetic heart valve comprising: an annular frame that is radially compressible and expandable between a radially compressed state and a radially expanded state, wherein the frame elongates in an axial direction when radially compressed from the radially expanded state to the radially compressed state; and a plurality of leaflets disposed inside of the frame and configured to permit blood to flow through the frame in a first direction and block the flow of blood in a second direction, opposite the first direction; wherein the leaflets are coupled to the frame such that when the frame is in the radially expanded state, the leaflets have slack in an axial direction, wherein when the frame is radially compressed to a partially radially compressed state between the radially expanded state and the radially compressed state, the leaflets are in a relaxed state, and wherein when the frame is in the radially compressed state, the leaflets are stretched in the axial direction.

Example 2: The prosthetic heart valve of any example herein, particularly example 1, wherein the frame comprises a plurality of struts pivotably connected to each other at a plurality of pivot joints, wherein the struts can pivot relative to each other when the frame is radially compressed and expanded.

Example 3: The prosthetic heart valve of any example herein, particularly any one of examples 1-2, wherein each leaflet comprises a cusp edge portion connected to the frame and two opposing commissure tabs, wherein each commissure tab is paired with an adjacent commissure tab of an adjacent leaflet to form a commissure, which is connected to the frame.

Example 4: The prosthetic heart valve of any example herein, particularly example 3, wherein each leaflet comprises two opposing neck regions, each extending from one of the commissure tabs to an adjacent end of the cusp edge portion, wherein the neck regions are unattached to the frame such that the neck regions can stretch in the axial direction when the frame is in the radially compressed state.

Example 5: The prosthetic heart valve of any example herein, particularly example 4, wherein neck regions define notches in the leaflets.

Example 6: The prosthetic heart valve of any example herein, particularly any one of examples 3-5, further comprising a plurality of actuators connected to the frame, wherein the actuators are configured to produce radial expansion and compression of the frame.

Example 7: The prosthetic heart valve of any example herein, particularly example 6, wherein each commissure is connected to one of the actuators.

Example 8: The prosthetic heart valve of any example herein, particularly any one of examples 3-7, further comprising a plurality of fabric skirts, each being sutured to the cusp edge portion of a corresponding leaflet and to the frame.

Example 9: The prosthetic heart valve of any example herein, particularly example 8, wherein each fabric skirt has opposing ends that are aligned with adjacent ends of the cusp edge portion of a corresponding leaflet.

Example 10: The prosthetic heart valve of any example herein, particularly any one of examples 8-9, further comprising a plurality of fabric reinforcing strips, each being sutured to the cusp edge portion of a corresponding leaflet on an opposite side of the cusp edge portion from the fabric skirt of the same leaflet.

Example 11: A prosthetic heart valve comprising: an annular frame that is radially compressible and expandable between a radially compressed state and a radially expanded state, wherein the frame elongates in an axial direction when radially compressed from the radially expanded state to the radially compressed state; and a leaflet assembly comprising a plurality of leaflets connected to each other at commissures of the leaflet assembly, wherein the leaflets are configured to permit blood to flow through the frame in a first direction and block the flow of blood in a second direction, opposite the first direction, wherein each commissure is coupled to the frame and each leaflet has a cusp edge portion that is coupled to the frame, and wherein each leaflet has two opposing neck regions unattached to the frame, each neck region extending from one of the commissures to an adjacent end of the cusp edge portion; wherein when the frame is in the radially expanded state, the leaflets have slack in an axial direction in the neck regions of the leaflets, wherein when the frame is radially compressed to a partially radially compressed state between the radially expanded state and the radially compressed state, the leaflets are in a relaxed state, and wherein when the frame is in the radially compressed state, the leaflets are stretched in the axial direction along the neck regions.

Example 12: The prosthetic heart valve of any example herein, particularly example 11, wherein neck regions define notches in the leaflets.

Example 13: The prosthetic heart valve of any example herein, particularly any one of examples 11-12, further comprising a plurality of actuators connected to the frame, wherein the actuators are configured to produce radial expansion and compression of the frame.

Example 14: The prosthetic heart valve of any example herein, particularly example 13, wherein each commissure is connected to one of the actuators.

Example 15: The prosthetic heart valve of any example herein, particularly any one of examples 11-14, further comprising a plurality of fabric skirts, each being sutured to the cusp edge portion of a corresponding leaflet and to struts of the frame.

Example 16: The prosthetic heart valve of any example herein, particularly any one of examples 11-15, wherein each fabric skirt has opposing ends that are aligned with adjacent ends of the cusp edge portion of a corresponding leaflet.

Example 17: The prosthetic heart valve of any example herein, particularly any one of examples 15-16, further comprising a plurality of fabric reinforcing strips, each being sutured to the cusp edge portion of a corresponding leaflet on an opposite side of the cusp edge portion from the fabric skirt of the same leaflet.

Example 18: A method of assembling a prosthetic heart valve, the method comprising: placing a plurality of leaflets in an annular frame that is radially compressible and expandable between a radially compressed state and a radially expanded state, wherein the frame elongates in an axial direction when radially compressed from the radially expanded state to the radially compressed state; placing the frame in a partially radially compressed state between the radially expanded state and the radially compressed state; and coupling the leaflets to the frame while the leaflets are in a relaxed state and the frame is in the partially radially compressed state such that when the frame is expanded to the radially expanded state, the leaflets have slack in an axial direction, and wherein when the frame is compressed to the radially compressed state, the leaflets are stretched in the axial direction.

Example 19: The method of any example herein, particularly example 18. wherein each leaflet comprises a cusp edge portion, two opposing commissure tabs, and two opposing neck regions, each neck region extending from one of the commissure tabs to an adjacent end of the cusp edge portion, wherein each commissure tab is paired with an adjacent commissure tab of an adjacent leaflet to form a commissure, and wherein coupling the leaflets to the frame comprises coupling the cusp edge portion of each leaflet to the frame and coupling each commissure to the frame such that the neck regions remain unattached to the frame.

Example 20: The method of any example herein, particularly example 19, wherein the cusp edge portion of cach leaflet is sutured to a skirt, which is connected to struts of the frame with sutures, each commissure is connected to a commissure support portion of the frame, and each neck region defines an axially extending gap between an adjacent commissure and an adjacent end of the cusp edge portion of a corresponding leaflet.

Example 21: The method of any example herein, particularly example 20, wherein the cusp edge portion of each leaflet is sutured to a discrete skirt.

Example 22: The method of any example herein, particularly example 20, wherein the cusp edge portion of each leaflet is sutured to the same skirt that extends an entire circumference of an inner surface of the frame.

Example 23: The method of any example herein, particularly any one of examples 20-22, wherein the cusp edge portion of each leaflet is sutured to a reinforcing strip opposite the skirt.

Example 24: A prosthetic heart valve comprising: an annular frame that is radially compressible and expandable between a radially compressed state and a radially expanded state, the frame comprising a plurality of interconnected struts; a plurality of leaflets connected to each other to form commissures, wherein the leaflets are configured to permit blood to flow through the frame in a first direction and block the flow of blood in a second direction, opposite the first direction, wherein each commissure is coupled to the frame and each leaflet has a cusp edge portion that is coupled to the frame, and wherein each leaflet has two opposing neck regions unattached to the frame, each neck region extending from one of the commissures to an adjacent end of the cusp edge portion; a plurality of discrete fabric skirts, each being sutured to the cusp edge portion of a leaflet on an outflow surface of the leaflet, wherein each fabric skirt has an extension portion extending beyond the cusp edge portion of the leaflet, the extension portion being connected to one or more of the struts of the frame with sutures; and a plurality of discrete fabric reinforcing strips, each being sutured to the cusp edge portion of a leaflet on an inflow surface of the leaflet.

Example 25: The prosthetic heart valve of any example herein, particularly example 24, wherein each of the discrete fabric skirts has opposite ends that are aligned with adjacent ends of the cusp edge portion of a corresponding leaflet.

Example 26: The prosthetic heart valve of any example herein, particularly any one of examples 24-25, wherein the leaflets are sized relative to the frame such that when the frame is in the radially expanded state, the leaflets have slack in an axial direction in the neck regions of the leaflets, wherein when the frame is radially compressed to a partially radially compressed state between the radially expanded state and the radially compressed state, the leaflets are in a relaxed state, and wherein when the frame is in the radially compressed state, the leaflets are stretched in the axial direction along the neck regions.

Example 27: The prosthetic heart valve of any example herein, particularly any one of examples 24-26, wherein neck regions define notches in the leaflets.

Example 28: The prosthetic heart valve of any example herein, particularly any one of examples 24-27, further comprising a plurality of actuators connected to the frame, wherein the actuators are configured to produce radial expansion and compression of the frame.

Example 29: The prosthetic heart valve of any example herein, particularly example 28, wherein each commissure is connected to one of the actuators.

Example 30: The prosthetic heart valve of any example herein, particularly any one of examples 24-29, wherein the plurality of struts comprises a plurality of inner struts and a plurality of outer struts pivotably connected to the inner struts at a plurality of pivot joints, wherein the struts can pivot relative to each other when the frame is radially compressed and expanded.

Example 31: The prosthetic heart valve of any example herein, particularly any one of examples 24-30, further comprising an outer fabric skirt extending around an outer surface of the frame.

Example 32: The prosthetic heart valve of any example herein, particularly example 31, wherein the extension portion of each discrete fabric skirt is stitched to the outer fabric skirt.

Example 33: A method of assembling a prosthetic heart valve, the method comprising: forming a plurality of leaflet sub-assemblies, each leaflet sub-assembly including a leaflet and a skirt, wherein each leaflet comprises a cusp edge portion, two opposing commissure tabs, and two opposing neck regions, each neck region extending from one of the commissure tabs to an adjacent end of the cusp edge portion. wherein each leaflet sub-assembly is formed by securing the skirt on an outflow side of the leaflet such that the skirt extends along the cusp edge portion; after forming each leaflet sub-assembly. forming a leaflet assembly by connecting each commissure tab of each leaflet sub-assembly with a commissure tab of another leaflet sub-assembly to form a commissure of the leaflet assembly; and after forming the leaflet assembly coupling the leaflet assembly to a frame of the prosthetic valve by connecting each commissure to a commissure support portion of the frame and connecting the skirt of each leaflet sub-assembly to struts of the frame, wherein the neck regions remain unattached to the frame.

Example 34: The method of any example herein, particularly example 33, wherein the skirt of each leaflet sub-assembly has opposing ends that are aligned with opposing ends of the cusp edge portion of the leaflet.

Example 35: The method of any example herein, particularly any one of examples 33-34, wherein forming each leaflet sub-assembly further comprises securing a reinforcing strip on an inflow side of the leaflet along the cusp edge portion.

Example 36: The method of any example herein, particularly example 35, wherein for each leaflet sub-assembly, the skirt and the reinforcing strip are secured to the cusp edge portion of the leaflet with stitches that extend through the skirt, the cusp edge portion of the leaflet, and the reinforcing strip.

Example 37: The method of any example herein, particularly any one of examples 33-36, wherein connecting the skirt of each leaflet sub-assembly comprises suturing the skirt to struts of the frame.

Example 38: The method of any example herein, particularly any one of examples 33-37, further comprising placing an outer skirt around an outer surface of the frame.

Example 39: The method of any example herein, particularly example 37, further comprising stitching the skirt of each leaflet sub-assembly to the outer skirt.

Example 40: The method of any example herein, particularly any one of examples 33-39, wherein neck regions define notches in the leaflets.

Example 41: The method of any example herein, particularly any one of examples 33-40, wherein the leaflet assembly is coupled to the frame while the while the leaflets arc in a relaxed state and the frame is in a partially radially compressed state such that when the frame is expanded to a radially expanded state, the leaflets have slack in an axial direction, and wherein when the frame is compressed to a radially compressed state, the leaflets are stretched in the axial direction.

Example 42: The method of any example herein. particularly any one of examples 33-41, wherein each commissure support is an actuator that is configured to produce radial expansion and compression of the frame and each commissure is connected to an actuator.

Example 43: A method of assembling a prosthetic heart valve, the method comprising: placing a plurality of leaflets in an annular frame that is radially compressible and expandable between a radially compressed state and a radially expanded state, wherein the frame elongates in an axial direction when radially compressed from the radially expanded state to the radially compressed state; placing the frame in a partially radially compressed state between the radially expanded state and the radially compressed state; and coupling the leaflets to the frame while the leaflets are in a relaxed state and the frame is in the partially radially compressed state such that when the frame is expanded to the radially expanded state, the leaflets are stretched in a widthwise direction.

Example 44: The method of any example herein, particularly example 44, wherein the leaflets are sized such that when the frame is expanded to the radially expanded state, the leaflets have slack in an axial direction, and wherein when the frame is compressed to the radially compressed state, the leaflets are stretched in the axial direction.

Example 45: The method of any example herein, particularly any one of examples 43-44, wherein each leaflet comprises a cusp edge portion, two opposing commissure tabs, and two opposing neck regions, each neck region extending from one of the commissure tabs to an adjacent end of the cusp edge portion, wherein each commissure tab is paired with an adjacent commissure tab of an adjacent leaflet to form a commissure, and wherein coupling the leaflets to the frame comprises coupling the cusp edge portion of each leaflet to the frame and coupling each commissure to the frame such that the neck regions remain unattached to the frame.

Example 46: The method of any example herein, particularly example 45, wherein the cusp edge portion of cach leaflet is sutured to a skirt, which is connected to struts of the frame with sutures, each commissure is connected to a commissure support portion of the frame, and each neck region defines an axially extending gap between an adjacent commissure and an adjacent end of the cusp edge portion of a corresponding leaflet.

Example 47: The method of any example herein, particularly example 46, wherein the cusp edge portion of each leaflet is sutured to a discrete skirt.

Example 48: The method of any example herein, particularly example 46, wherein the cusp edge portion of each leaflet is sutured to the same skirt that extends an entire circumference of an inner surface of the frame.

Example 49: The method of any example herein, particularly any one of examples 45-48, wherein the cusp edge portion of each leaflet is sutured to a reinforcing strip opposite the skirt.

In view of the many possible embodiments to which the principles of the disclosed invention may be applied, it should be recognized that the illustrated embodiments are only preferred examples of the invention and should not be taken as limiting the scope of the invention. Rather, the scope of the invention is defined by the following claims. We therefore claim as our invention all that comes within the scope and spirit of these claims. The following is a list of aspects of the disclosure:
1. A prosthetic heart valve comprising:
   an annular frame that is radially compressible and expandable between a radially compressed state and a radially expanded state, wherein the frame elongates in an axial direction when radially compressed from the radially expanded state to the radially compressed state; and
   a plurality of leaflets disposed inside of the frame and configured to permit blood to flow through the frame in a first direction and block the flow of blood in a second direction, opposite the first direction;
   wherein the leaflets are coupled to the frame such that when the frame is in the radially expanded state, the leaflets have slack in an axial direction, wherein when the frame is radially compressed to a partially radially compressed state between the radially expanded state and the radially compressed state, the leaflets are in a relaxed state, and wherein when the frame is in the radially compressed state, the leaflets are stretched in the axial direction.
2. The prosthetic heart valve of clause 1, wherein the frame comprises a plurality of struts pivotably connected to each other at a plurality of pivot joints, wherein the struts can pivot relative to each other when the frame is radially compressed and expanded.
3. The prosthetic heart valve of any previous clause, wherein each leaflet comprises a cusp edge portion connected to the frame and two opposing commissure tabs, wherein each commissure tab is paired with an adjacent commissure tab of an adjacent leaflet to form a commissure, which is connected to the frame.
4. The prosthetic heart valve of clause 3, wherein each leaflet comprises two opposing neck regions, each extending from one of the commissure tabs to an adjacent end of the cusp edge portion, wherein the neck regions are unattached to the frame such that the neck regions can stretch in the axial direction when the frame is in the radially compressed state.
5. The prosthetic heart valve of clause 4, wherein neck regions define notches in the leaflets.
6. The prosthetic heart valve of any of clauses 3-5, further comprising a plurality of actuators connected to the frame, wherein the actuators are configured to produce radial expansion and compression of the frame.
7. The prosthetic heart valve of clause 6, wherein each commissure is connected to one of the actuators.
8. The prosthetic heart valve of any of clauses 3-7, further comprising a plurality of fabric skirts, each being sutured to the cusp edge portion of a corresponding leaflet and to the frame.
9. The prosthetic heart valve of clause 8, wherein each fabric skirt has opposing ends that are aligned with adjacent ends of the cusp edge portion of a corresponding leaflet.
10. The prosthetic heart valve of any of clauses 8-9, further comprising a plurality of fabric reinforcing strips, each being sutured to the cusp edge portion of a corresponding leaflet on an opposite side of the cusp edge portion from the fabric skirt of the same leaflet.
11. A method of assembling a prosthetic heart valve, the method comprising:
   placing a plurality of leaflets in an annular frame that is radially compressible and expandable between a radially compressed state and a radially expanded state, wherein the frame elongates in an axial direction when radially compressed from the radially expanded state to the radially compressed state;
   placing the frame in a partially radially compressed state between the radially expanded state and the radially compressed state; and
   coupling the leaflets to the frame while the leaflets are in a relaxed state and the frame is in the partially radially compressed state such that when the frame is expanded to the radially expanded state, the leaflets have slack in an axial direction, and wherein when the frame is compressed to the radially compressed state, the leaflets are stretched in the axial direction.
12. The method of clause 11, wherein each leaflet comprises a cusp edge portion, two opposing commissure tabs, and two opposing neck regions, each neck region extending from one of the commissure tabs to an adjacent end of the cusp edge portion, wherein each commissure tab is paired with an adjacent commissure tab of an adjacent leaflet to form a commissure, and wherein coupling the leaflets to the frame comprises coupling the cusp edge portion of each leaflet to the frame and coupling each commissure to the frame such that the neck regions remain unattached to the frame.
13. The method of clause 12, wherein the cusp edge portion of each leaflet is sutured to a skirt, which is connected to struts of the frame with sutures, each commissure is connected to a commissure support portion of the frame, and each neck region defines an axially extending gap between an adjacent commissure and an adjacent end of the cusp edge portion of a corresponding leaflet.
14. The method of clause 13, wherein the cusp edge portion of each leaflet is sutured to a discrete skirt.
15. The method of clause 13, wherein the cusp edge portion of each leaflet is sutured to the same skirt that extends an entire circumference of an inner surface of the frame.
16. The method of any of clauses 13-15, wherein the cusp edge portion of each leaflet is sutured to a reinforcing strip opposite the skirt.
17. A prosthetic heart valve comprising:
   an annular frame that is radially compressible and expandable between a radially compressed state and a radially expanded state, the frame comprising a plurality of interconnected struts;
   a plurality of leaflets connected to each other to form commissures, wherein the leaflets are configured to permit blood to flow through the frame in a first direction and block the flow of blood in a second direction, opposite the first direction, wherein each commissure is coupled to the frame and each leaflet has a cusp edge portion that is coupled to the frame, and wherein each leaflet has two opposing neck regions unattached to the frame, each neck region extending from one of the commissures to an adjacent end of the cusp edge portion;
   a plurality of discrete fabric skirts, each being sutured to the cusp edge portion of a leaflet on an outflow surface of the leaflet, wherein each fabric skirt has an extension portion extending beyond the cusp edge portion of the leaflet, the extension portion being connected to one or more of the struts of the frame with sutures; and
   a plurality of discrete fabric reinforcing strips, each being sutured to the cusp edge portion of a leaflet on an inflow surface of the leaflet.
18. The prosthetic heart valve of clause 17, wherein each of the discrete fabric skirts has opposite ends that are aligned with adjacent ends of the cusp edge portion of a corresponding leaflet.
19. The prosthetic heart valve of any of clauses 17-18, wherein the leaflets are sized relative to the frame such that when the frame is in the radially expanded state, the leaflets have slack in an axial direction in the neck regions of the leaflets, wherein when the frame is radially compressed to a partially radially compressed state between the radially expanded state and the radially compressed state, the leaflets are in a relaxed state, and wherein when the frame is in the radially compressed state, the leaflets are stretched in the axial direction along the neck regions.
20. The prosthetic heart valve of any of clauses 17-19, wherein neck regions define notches in the leaflets.
21. The prosthetic heart valve of any of clauses 17-20, further comprising a plurality of actuators connected to the frame, wherein the actuators are configured to produce radial expansion and compression of the frame.
22. The prosthetic heart valve of clause 21, wherein each commissure is connected to one of the actuators.
23. The prosthetic heart valve of any of clauses 17-22, wherein the plurality of struts comprises a plurality of inner struts and a plurality of outer struts pivotably connected to the inner struts at a plurality of pivot joints, wherein the struts can pivot relative to each other when the frame is radially compressed and expanded.
24. The prosthetic heart valve of any of clauses 17-23, further comprising an outer fabric skirt extending around an outer surface of the frame.
25. The prosthetic heart valve of clause 24, wherein the extension portion of each discrete fabric skirt is stitched to the outer fabric skirt.

## Claims

1. A prosthetic heart valve comprising:
an annular frame that is radially compressible and expandable between a radially compressed state and a radially expanded state, the frame comprising a plurality of interconnected struts;
a plurality of leaflets connected to each other to form commissures, wherein the leaflets are configured to permit blood to flow through the frame in a first direction and block the flow of blood in a second direction, opposite the first direction, wherein each commissure is coupled to the frame and each leaflet has a cusp edge portion that is coupled to the frame, and wherein each leaflet has two opposing neck regions unattached to the frame, each neck region extending from one of the commissures to an adjacent end of the cusp edge portion;
a plurality of discrete fabric skirts, each being sutured to the cusp edge portion of a leaflet on an outflow surface of the leaflet, wherein each fabric skirt has an extension portion extending beyond the cusp edge portion of the leaflet, the extension portion being connected to one or more of the struts of the frame with sutures; and
a plurality of discrete fabric reinforcing strips, each being sutured to the cusp edge portion of a leaflet on an inflow surface of the leaflet.

2. The prosthetic heart valve of claim 1, wherein each of the discrete fabric skirts has opposite ends that are aligned with adjacent ends of the cusp edge portion of a corresponding leaflet.

3. The prosthetic heart valve of any preceding claim, wherein the leaflets are sized relative to the frame such that when the frame is in the radially expanded state, the leaflets have slack in an axial direction in the neck regions of the leaflets, wherein when the frame is radially compressed to a partially radially compressed state between the radially expanded state and the radially compressed state, the leaflets are in a relaxed state, and wherein when the frame is in the radially compressed state, the leaflets are stretched in the axial direction along the neck regions.

4. The prosthetic heart valve of any preceding claim, wherein neck regions define notches in the leaflets.

5. The prosthetic heart valve of any preceding claim, further comprising a plurality of actuators connected to the frame, wherein the actuators are configured to produce radial expansion and compression of the frame.

6. The prosthetic heart valve of claim 5, wherein each commissure is connected to one of the actuators.

7. The prosthetic heart valve of any preceding claim, wherein the plurality of struts comprises a plurality of inner struts and a plurality of outer struts pivotably connected to the inner struts at a plurality of pivot joints, wherein the struts can pivot relative to each other when the frame is radially compressed and expanded.

8. The prosthetic heart valve of any preceding claim, further comprising an outer fabric skirt extending around an outer surface of the frame.

9. The prosthetic heart valve of claim 8, wherein the extension portion of each discrete fabric skirt is stitched to the outer fabric skirt.
